Europäisches Patentamt

(19) European Patent Office

Office européen des brevets

(11) **EP 1 322 218 B1**

(12) **EUROPEAN PATENT SPECIFICATION**

(45) Date of publication and mention
of the grant of the patent:
**11.05.2005 Bulletin 2005/19**

(21) Application number: **01974456.4**

(22) Date of filing: **05.10.2001**

(51) Int Cl.⁷: **A61B 5/00**

(86) International application number:
**PCT/GB2001/004438**

(87) International publication number:
**WO 2002/030276 (18.04.2002 Gazette 2002/16)**

(54) **APPARATUS AND METHOD FOR MEASUREMENT OF BIOLOGICAL STRUCTURES**

GERÄT UND VERFAHREN ZUR MESSUNG VON BIOLOGISCHEN STRUKTUREN

DISPOSITIF ET METHODE DE MESURES DE STRUCTURES BIOLOGIQUES

(84) Designated Contracting States:
**AT BE CH CY DE DK ES FI FR GB GR IE IT LI LU MC NL PT SE TR**

(30) Priority: **07.10.2000 GB 0024641**

(43) Date of publication of application:
**02.07.2003 Bulletin 2003/27**

(73) Proprietor: **The Court of Glasgow Caledonian University**
**Glasgow G4 0BA (GB)**

(72) Inventors:
• **HILLIER, Christopher**
**Clarkston Glasgow G76 7HN (GB)**

• **BUNTON, David**
**Cambuslang Glasgow G72 7GH (GB)**
• **COATS, Paul**
**Hamilton, South Lanarkshire ML3 7HD (GB)**
• **SMITH, Peter**
**Loughborough LE11 3JR (GB)**

(74) Representative: **Main, Malcolm Charles et al**
**Murgitroyd & Company**
**Scotland House**
**165-169 Scotland Street**
**Glasgow G5 8PL (GB)**

(56) References cited:
**WO-A-98/44841          US-A- 5 158 090**
**US-A- 5 846 188          US-A- 5 986 770**

**Description**

The present invention relates to an apparatus and to a method for the measurement of dimensions of biological structures. In particular, but not exclusively, the invention relates to the measurement of changes over time in the physical dimensions of biological structures.

The measurement of the size of biological structures, such as arteries or muscles, has a number of applications in the laboratory. In particular, it is often desirable to investigate contraction or relaxation of blood vessels and the like in response to hormones, agonists, or potential drugs.

Typically, such investigations are conducted with use of a myograph, that is, a device for measurement of muscle characteristics. wire myography involves mounting the vessel under study onto a pair of steel wires, which are secured to supports. Mounting the vessel is a difficult, time-consuming technique which requires considerable practice and often results in damage to the vessel, mainly because of the difficulty in aligning the wires with the lumen of the vessel. One support is attached to a micrometer, allowing control of vessel circumference, while the other support is attached to a force transducer, for measurements of tension development. The whole assembly is maintained in a chamber containing physiological salt solution, to which various test substances may be added to assess the vessel response. However, the quality of the information obtained from a wire myograph is limited because the vessel is not stretched in a manner that reflects the situation *in vivo.*

An alternative technique is pressure myography, where vessels are cannulated at both ends and examined under pressure.

Pressure myography creates a situation closer to that found *in vivo;* however, mounting vessels onto cannula is a difficult, time-consuming procedure.

Diameter and length of vessels under study may also be determined visually, either by an experienced experimenter under the microscope, or automatically by means of a computing device running image analysis software. However, such software must be relatively sophisticated to accurately image and analyse the dimensions of the vessel under study and such image analysis is difficult to apply to the internal diameter of vessels.

Perfusion myography requires substantial amounts of expensive equipment yet the number of experiments that can be conducted is extremely low.

Therefore, the use of transducers and imaging technologies are not conductive to conducting large numbers of experiments because of three fundamental disadvantages. Firstly, the tissue must be 'mounted' onto the measurement apparatus. This involves skilled technicians or scientists and can take valuable time. Secondly, a lack of automation in these systems means that they must be supervised and managed throughout the assay by a skilled technician. Thirdly, the systems are very expensive. These fundamental problems represent 'barriers to entry' that limit usage of such systems.

Document US-A-5 846 188 discloses a method and an apparatus for detecting changes in physical dimensions of biological structures, the apparatus comprising at least one light source, a light detector, means for locating a biological structure between the source and the detector, and data processing means for recording and analysing detected light which has been transmitted by the biological structure to detect and analyse changes in characteristics of the transmitted light to determine changes in the physical dimensions of the biological structure.

It is among the objects of embodiments of the present invention to obviate or alleviate these and other disadvantages of known myography techniques and systems.

According to a first aspect of the present invention, there is provided a method as defined in claim 1.

The present invention therefore makes use of the differing degree of absorption of light by biological structures with different diameters and thicknesses. Further, the present invention also allows detection of changes in the internal diameter of a tube structure, since this will affect the thickness and hence absorption of the structure.

Preferably, the method is used to measure a biological tube structure; for example, a blood vessel or the like.

Preferably, the detection of transmitted light comprises the step of detecting the intensity of transmitted light across substantially the whole diameter of the biological structure. It has been found that'such a relatively crude measurement technique in fact provides sufficient information to determine a change in the dimensions of the structure from a change in the absorption of the structure. There is thus no requirement to obtain a detailed optical image, nor to obtain images of many different points across the structure, in contrast to known myography techniques. This therefore provides advantages in terms of information gathering and processing.

Preferably, the method further comprises the step of repeating the illumination, detection, and comparison steps with light of a second wavelength. This may itself be performed as many times as desired, with further wavelengths. The use of additional wavelengths of light can provide additional information regarding the biological structure, due to the differing transmissivity of such structures at different wavelengths. Preferably, the wavelengths are selected such that in one of the wavelengths the biological structure is essentially opaque, whilst in another of the wavelengths the biological structure is semi-transparent. It will be readily apparent to the skilled person that with a general knowledge of the tissue being studied, it will be possible to select wavelengths such that some are highly absorbing while others provide contrast between paths of large and small optical density. The illumination, detection and comparison steps with second or subsequent wavelengths may be performed subsequently to the steps conducted with the first wavelength; alternatively, the steps may be performed simultaneously. Separate performance of the steps ensures that the information from each wavelength is kept separate; if the steps are performed simultaneously, it is preferred that the method further includes the step of distinguishing measured intensities of each wavelength of light used. For example, separate detectors may be used, or the detected combined intensity may be subjected to post-processing to separate the various wavelength components.

Preferably, the reference light intensity corresponds to the intensity of light measured when no biological sample is illuminated; that is, a blank reference measurement. This allows account to be taken of any features of the system used for making the measurements which affect the light wavelengths used. Alternatively, the reference light intensity may correspond to the measured intensity of light transmitted by the biological sample in a different physical state. This allows measurements of the change in transmissivity of a biological structure over time to be obtained. Of course, a combination of the two reference intensities may be used, to provide both an absolute and a relative measurement of transmitted light.

According to a second aspect of the present invention, there is provided a method of measuring changes in the physical dimensions of a biological structure over time, the method comprising the steps of:

illuminating a biological structure at first and second time points with light of a first wavelength;

detecting the intensity of light transmitted by the biological structure at said first and second time points; and

comparing the intensity of transmitted light at the first time point with the intensity of transmitted light at the second time point to determine a change in the intensity of transmitted light, said intensity change being indicative of a change in physical dimensions of the biological structure.

The method may further comprise the step of altering the local environment of the biological structure between said first and second time points. For example, this may include addition or removal of agonists, hormones, potential drugs, test samples and the like to the vicinity of the biological structure, or direct physical stimulation of the structure. The method may also include the step of transfecting the biological structure with exogenous nucleic acid; this may be of use in determining the effect of a gene or similar nucleic acid structure, or the potential stability of a transfection vector or of the biological structure for transfection. Alternatively, or in addition, peptide or protein structures may be added to the biological structure, to determine the effects of such peptides or proteins directly, without the requirement for expression of a transfected gene.

Formulae for use in the methods of the invention are described in detail with reference to the examples of the invention. The invention also provides use of these equations in the measurement of physical changes in tubular biological structure.

According to a further aspect of the present invention, there is provided an apparatus as defined in claim 15.

The light source may generate light of a single wavelength, or of a plurality of wavelengths. A number of distinct light sources may alternatively be provided.

The light detector may comprise, for example, a CCD device, a photoresistor, a photodiode, or the like. The detector may be capable of detecting light of only a single wavelength or of a plurality of wavelengths. Where the detector can detect a plurality of wavelengths of light, the apparatus further comprises means for separating detected light of multiple wavelengths: this may comprise post-detection means, such as a data processing means; or may comprise pre-detection means, such as means for generating light of a single wavelength at a time, or filter means for allowing only light of a single wavelength to reach the detector.

The apparatus may further comprise means for aligning generated and detected light. Preferably, this comprises aligned optical fibres, at least one of which carries generated light, and at least one of which carries detected light. The fibres are connected at one end to a light source or light detector, as appropriate, with the other ends of the fibres being in opposed alignment, such that light leaving the source fibre can enter the detection fibre. The apparatus may further comprise a plastics or similar optical fibre holder, arranged to hold optical fibres in oppositely aligned relationship. The holder may conveniently comprise a bifurcated tube, the tips of each fork of which are in alignment; in use, an optical fibre may be inserted into each fork of the holder, with the free ends of the fibres being coterminous with the ends of the forks. This ensures the optical fibres are aligned correctly.

The means for locating a biological structure may comprise a wire holder, a cannula, or the like.

Preferably, the locating means comprises a plastics tube bearing an external circumferential groove; in use, a biological tube structure may be located over the plastic tube, and secured thereto by means of tying a fastener around the groove. Conveniently, the tube is of polypropylene.

Preferably, the apparatus further comprises means for subjecting the biological structure to fluids and the like. Preferably, this comprises a chamber having a fluid inlet and a fluid outlet, in which the biological structure is located. The apparatus may further comprise one or more fluid reservoirs, for storing circulatory fluid. Conveniently, the chamber comprises a fluid pump, for circulating fluid within the chamber, or into and out of the chamber. Conveniently also, the pump may be arranged to maintain the chamber at substantially physiological fluid pressure. The apparatus may still further comprise means for introducing substances to the biological structure; for example, a syringe may be used to introduce a test substance to the circulating fluid. Preferably, a plurality of chambers are provided, enabling a plurality of biological structure samples to be studied simultaneously.

Preferably, the data processing means comprises a computer.

Following dissection, isolated tissue can be difficult to manipulate as it is flaccid in nature. This is especially true with respect to the accurate positioning of the tissue with regard to the locating means, which may comprise a wire holder, a cannula or the like. To obviate this disadvantage there is provided a method of immobilising and positioning the tissue that ensures alignment with the locating device, the method comprising placing the tissue upon a layer comprising one or more proteins selected from the following: collagen, elastin, gelatin, fibronectin, fibrinogen, vitronectin.

The method may further comprise the step of first placing the protein layer upon a polypropylene or polyethylene surface. Preferably, the plastic surface comprises the inner aspect of half of a conical funnel, ensuring a concave surface upon which to place the tissue. Two such funnels are then placed in opposition upon a rigid bath, with the mouths of the funnels facing each other. One funnel is fixed as part of the bath, the second funnel is placed on the bath, preferably attached to a rail that allows movement towards or away from the first funnel in the horizontal plane.

Further, the accurate positioning of the tissue with respect to the chosen locating device is ensured by placing one end of the tissue in the neck of the fixed conical funnel at a point that equals the external diameter of the tissue. The second funnel is moved along the horizontal plane until the end of the tissue in the neck of the conical funnel again lies above the point in the funnel that equals the external diameter of the tissue. The tissue is then placed on the protein layer of the moveable second funnel.

The locating device can be guided firstly through the narrow portion of the funnel and then through the immobilised tissue.

The means for locating the biological structure in the apparatus of the invention may include a layer of protein to immobilise the structure.

Typically the protein may be or may be derived from collagen, elastin, gelatin, fibronectin, fibrinogen, vitronectin or any combination thereof.

The invention also provides the use of at least one protein chosen from collagen, elastin, gelatin, fibronectin, fibrinogen and vitronectin to support biological tissue in testing apparatus.

The apparatus may further comprise temperature regulation means, for regulating the temperature of a biological sample.

These and other aspects of the present invention will now be described by way of example only and with reference to the accompanying drawings, in which:

Fig. 1 shows a schematic diagram of an implementation of the method of measuring biological structures of one aspect of the present invention;

Fig. 2 shows a hypothetical example of the intensity of light of two different wavelengths transmitted through a hypothetical biological structure;

Fig. 3 shows a schematic diagram of an apparatus for measurement of biological structures in accordance with a further embodiment of the present invention;

Fig. 4 shows a fibre optic holder as may be used with an apparatus for measurement of biological structures in accordance with an embodiment of the present invention;

Fig. 5 shows a biological structure mounting means as may be used with an apparatus in accordance with an embodiment of the present invention;

Fig. 6 shows an apparatus for measurement of biological structures in accordance with an embodiment of the present invention;

Fig. 7 shows a graph of the percentage change in lumen diameter of a blood vessel in response to differing concentrations of an agonist, as determined with the apparatus of Fig. 6;

Fig. 8 shows the geometry of a vessel under tension in a myograph, as described in more detail with reference to Experiment 2 below;

Fig. 9 is a schematic diagram of the experimental apparatus for absorption spectroscopy using the Mulvany myograph, as described in Experiment 2 below;

Fig. 10 shows transmission spectra obtained using the adapted Mulvany myograph for normal (N1, N2: BP=135.4 mmHg, media thickness=25.32μm) and hypertensive (H1, H2: BP=231 mmHg, media thickness=46.7μm) vessels under tension produced by a linear stretch of 250 μm (N1, H1) and 300 μm (N2, H2). Spectra are obtained from paired specimens;

Fig. 11 shows the fitted averaged normalised transmission spectrum of a control vessel under a linear stretch of 350 μm. Data points are represented by circles and the continuous line is given by;

$$T = \frac{0.39}{1+\exp(-0.057[\lambda-412])} + \frac{0.098}{1+\exp(-0.179[\lambda-753])}$$

Fig. 12 is a table showing blood pressures (tail-cuff) and morphological characteristics of mesentery arteries (mean ± sem);

Fig. 13 is a matrix of correlation coefficients. Data derived from all groups (4 week, 8 week clipped and control.) Total number of spectra analysed = 659 (*p<0.001, normotensive vs. hypertensive); and

Fig. 14 represents a locating means to position tissue for testing in apparatus of the invention.

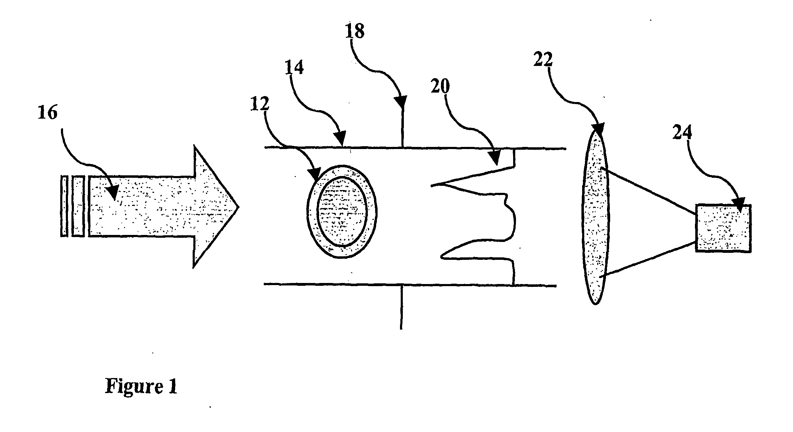

Referring first of all to Fig. 1, this shows a schematic arrangement for measuring biological structures, in accordance with one embodiment of the present invention. A biological tube structure 12, such as a blood vessel, is suspended in an enclosure 14, such that the blood vessel 12 may be illuminated by a light source 16. An aperture 18 allows only a portion of the light to be transmitted. The light source 16 is a combination of a number of spectral components; each spectral component will generate an intensity profile 20 of light transmitted by the blood vessel 12, whose characteristics will be a function of the blood vessel geometry and optical properties. A focussing lens 22 and a detector 24 are used to obtain a single point intensity measurement of the transmitted light for each spectral component. This single measurement is essentially the integrated intensity profile for the spectral component.

A set of measurements consists of extinction values (integrated intensity profiles) of the tissue-transmitted intensity for each distinct spectral component, these are labelled $\varepsilon_i$. A reference set of measurements $\varepsilon_{0i}$ are taken without tissue present.

The spectral components are chosen so that in one (or some) of the components the tissue is essentially opaque whilst in another (others) the tissue is semi-transparent. The transition between these conditions is actually quite narrow, because the light absorption increases (essentially) exponentially with optical density. Thus with a general knowledge of the tissue being studied it is always possible to select spectral components that are highly absorbing and ones that provide contrast between paths of large and small optical density. This property is used in the standard perfusion myograph to provide imaging contrast between the vessel lumen and vessel wall.

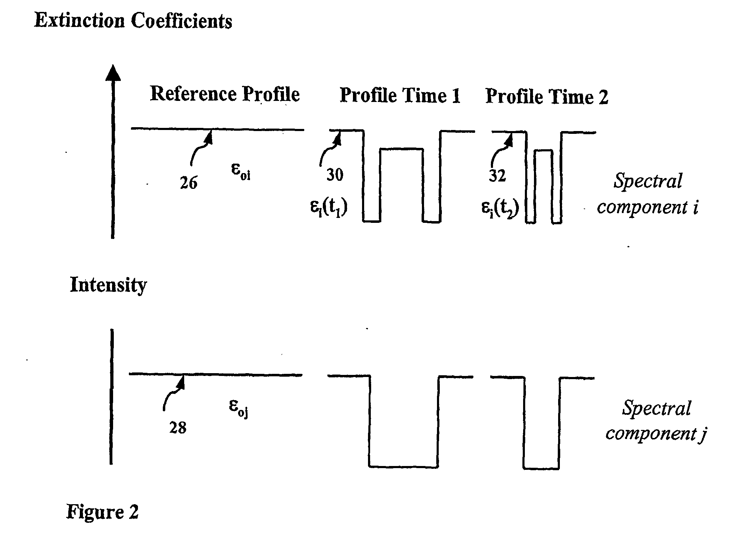

Figure 2 shows a series of hypothetical representative intensity measurements for a blood vessel model with two spectral components, i and j. For each spectral component there is shown a reference measurement $\varepsilon_{0i}$, $\varepsilon_{0j}$, denoted by reference numerals 26, 28, and a.pair of extinction measurements $\varepsilon_i (t_1)$, $\varepsilon_i (t_2)$ (reference numerals 30, 32) taken at successive times. It will be seen that the characteristics of the vessel alter from time $t_1$ to time $t_2$ (for example, a hormone is added to the system causing the vessel to contract), and that the vessel is essentially opaque for spectral component j, so that no information can be derived concerning the vessel lumen from this component alone. Because these measurements are all derived from a fixed aperture, a simple model of the relationship between the extinction value and the profile may be written as:

$$\varepsilon_{0i} = MI_i$$

$$\varepsilon_i(t) = (M - V(t))I_i + (V(t) - L(t))I_i\alpha_{Vi} + L(t)I_i\alpha_{Li}$$

where we have assumed that the illumination pattern is uniform with intensity per unit length $I_i$ and M is a measure of the aperture size. The variable vessel and lumen sizes are represented by $(V(t),L(t))$ respectively and the light absorption coefficients in the vessel and lumen are represented by $(\alpha_{Vi},\alpha_{Li})$. It is convenient to form the normalised extinction coefficients as follows, where the normalised values are denoted by a hat over the variable:

$$\hat{\varepsilon}_i(t) = \frac{\varepsilon_i(t)}{\varepsilon_{0i}} = 1-(\hat{V}(t)-\hat{L}(t))\alpha_{Vi}+\hat{L}(t)\alpha_{Li}$$

Two instances of this equation (i.e. for two spectral components) can be solved for the vessel and lumen sizes, providing the tissue optical proprieties are known (or can be calibrated). Further simplification can be achieved when the vessel wall is essentially opaque for certain selected spectral components, such as spectral component j, in which case the normalised extinction coefficient reduces to:

$$\hat{\varepsilon}_j(t)=1-\hat{V}(t)$$

and hence the lumen size may be estimated from:

$$\hat{L}(t) = \frac{\hat{\varepsilon}_j(t)(1-\alpha_{Vi})+\alpha_{Vi}-\hat{\varepsilon}_i(t)}{\alpha_{Vi}-\alpha_{Li}}$$

With careful choice of spectral components, as selected by a person of skill in the art, the following condition may apply

$$\alpha_{Vi}\gg\alpha_{Li}$$

And hence

$$\hat{L}(t) \cong 1 + \hat{\varepsilon}_j(t)(\frac{1}{\alpha_{Vi}}-1) - \frac{1}{\alpha_{Vi}}\hat{\varepsilon}_i(t)$$

Many more models of the tissue geometry and associated extinction profiles could be envisaged. The one considered here is only presented as an example. It is intended to illustrate how a relationship exists between the vessel lumen and the measured normalised extinction coefficients of at least two contrasting spectral components. Although the relationship derived here is a linear one, in principle the relationship can be non-linear. In a real system this relationship can be explored by means of calibration with respect to tissue phantoms (that is, a material constructed to mimic the geometrical and optical properties of a real tissue sample; for example, a cylinder constructed of optical resin embedded with fine sand) that mimic the geometry and scattering properties of a range of vessels. Such phantoms are straightforward to construct.

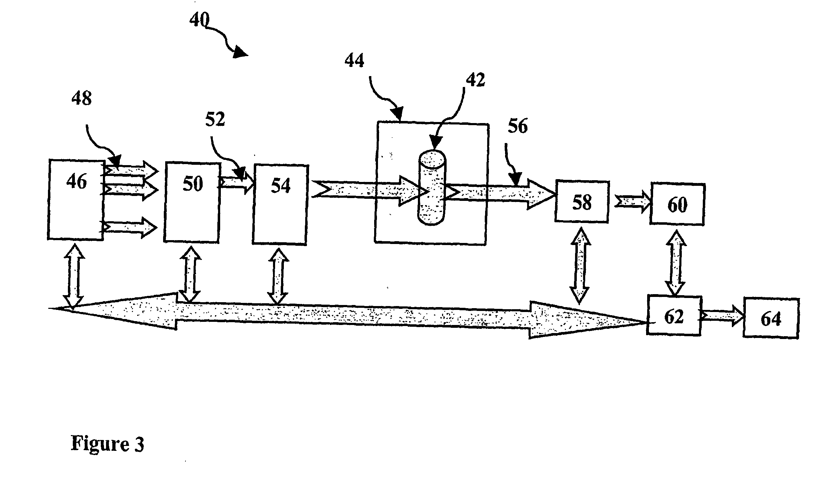

Referring now to Figure 3, this shows in broad outline an apparatus 40 for measurement of biological structures such as a blood vessel 42. The apparatus 40 includes a chamber 44 in which the blood vessel 42 is mounted and supplied with suitable media for maintaining the condition of the vessel 42. A light source 46 generates a range of spectral components 48, which components 48 are passed through a lens 50 which collimates and aligns the distinct components into a single light field 52. The light field 52 is then directed by means of a fibre optic arrangement 54 to illuminate a suitable area of the chamber 44 and vessel 42. Light which is transmitted 56 by the vessel 42 is received and directed by means of a further optical fibre arrangement 58 to a suitable light detector 60 which provides an integrated intensity profile of the transmitted light. The intensity data is passed to a data processor 62, such as a personal computer, which can also be arranged to receive data from and pass data to the other components of the apparatus 40, and to control the operation of the apparatus. The PC 62 is also connected to an output device 64, such as a monitor or a printer, for generating a record of experimental data.

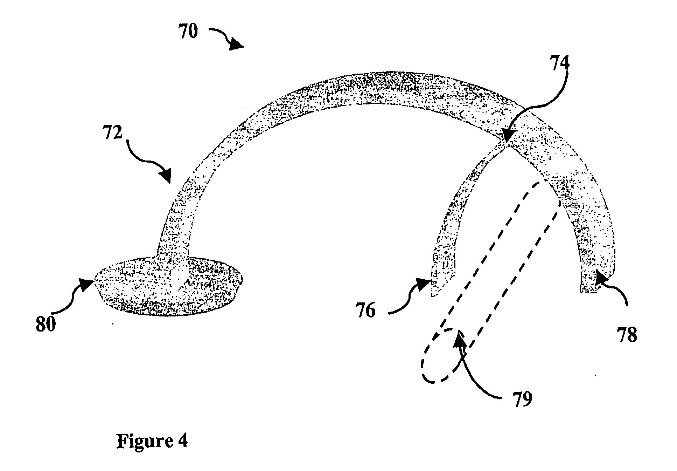

Various components of the apparatus 40 will now be described in more detail. Figure 4 shows an optical fibre holder 70 which may be used with the apparatus 40 to ensure appropriate direction of illuminating and transmitted light (by means of optical fibre arrangements 54 and 58 on Figure 3). The holder 70 comprises tubular moulded plastic 72 forming an inverted cup shape with a bifurcation point 74 resulting in two channel portions 76, 78, the free ends of which are opposed. The holder 70 is mounted on a circular swivel mount 80, allowing the holder to be easily moved into and out of position. In use, two optical fibres, one serving as a transmitter, and one as a receiver, are inserted into the tube 72 from the swivel mount 80. The transmitter fibre is fed along the tube to the left channel portion 76, while the receiving fibre is fed to the right channel portion 78. The ends of the fibre are adjusted to sit flush with the ends of the channel portions 76, 78, to ensure that light leaving the transmitter fibre can be detected by the receiving fibre. The other ends of the fibres are connected to the appropriate transmitter or detector parts of the apparatus. In use, the holder 70 is placed so that each channel portion 76, 78 is on either side of a biological structure to be measured.

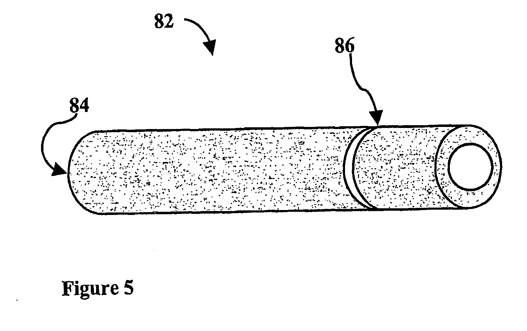

The biological structure, if it is a tube structure, may be supported between the portions of the holder by means of a tissue mount as shown in Figure 5. The tissue mount 82 comprises a polypropylene tube 84 with parallel walls and an external diameter roughly one tenth of the length, and an internal diameter approximately four-fifths of the external diameter. At a distance from one end generally equal to the external diameter of the tube, a 20mm groove 86 is present. To manufacture the tissue mount 82, standard polypropylene tubing (Camlab, Cambridge, UK) is cut to appropriate lengths and mounted on a microlathe. A micromanipulator holding a heated surgical needle (28 gauge) is slowly advanced towards the turning plastic tube, cutting a 20mm circumferential groove in the tubing. To mount a vessel on the mount 82, a section of vessel under study is pulled onto the mount until the edge of the vessel passes the groove 86. A small plastic tie is applied to the vessel and tightened gently over the groove to secure the vessel on the mount 82.

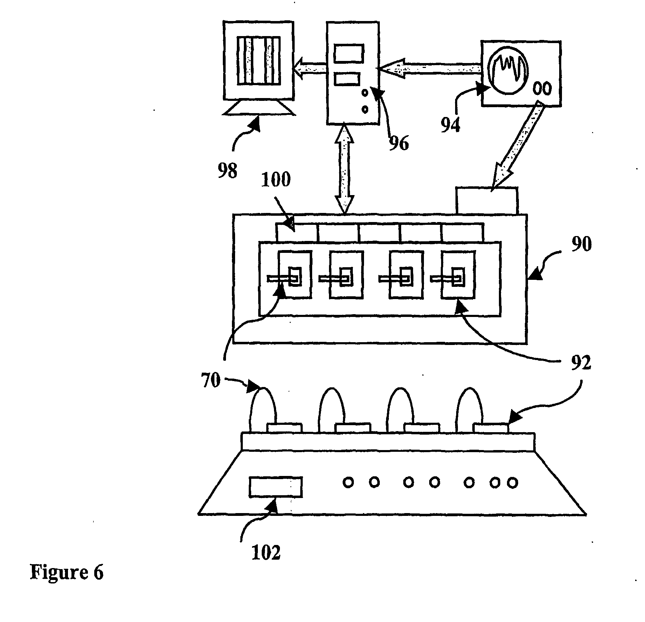

Figure 6 illustrates a further apparatus 90 for measurement of biological structures according to an embodiment of the invention. The apparatus 90 includes four chambers 92 in which an experimental sample can be located, each of which is provided with an optical fibre holder 70 as described.

The apparatus includes an oscilloscope 94, for monitoring the intensity of transmitted light, and is connected to a personal computer 96 with monitor 98 for analysing and displaying experimental data. The apparatus 90 also has multiple fluid reservoirs 100 for storing and dispensing media or other substances for use in experimental procedures, and a multi-channel cassette pump (not shown in the interest of clarity) for fluid pumping.

To prepare the apparatus 90 for experimental procedures, tissue mounts as described are attached to each of two stainless steel pipes in each chamber 92. A section of isolated blood vessel (length range 8-16mm; diameter range 100-1000μm) is pulled onto the proximal cannula until 2mm of vessel surrounds the tissue mount. The vessel is secured by means of a plastic tie, as described.

Prior to the study of the tissue, the optical fibre holder 70 is swivelled across and positioned across the tissue so that the end faces of the optical fibres are in apposition and the light emitted is directly perpendicular to the longitudinal axis of the tissue (as shown in chain dotted outline 79 in Figure 4).

The fluid reservoirs 100, connected to perfusion loops by a system of tubing and controlled by solenoid valves are filled with the appropriate solutions prior to commencing the experimental protocol. Generally, reservoirs will contain rinsing solution and drugs. Filling of the reservoir is done manually via filling hole prior to each study.

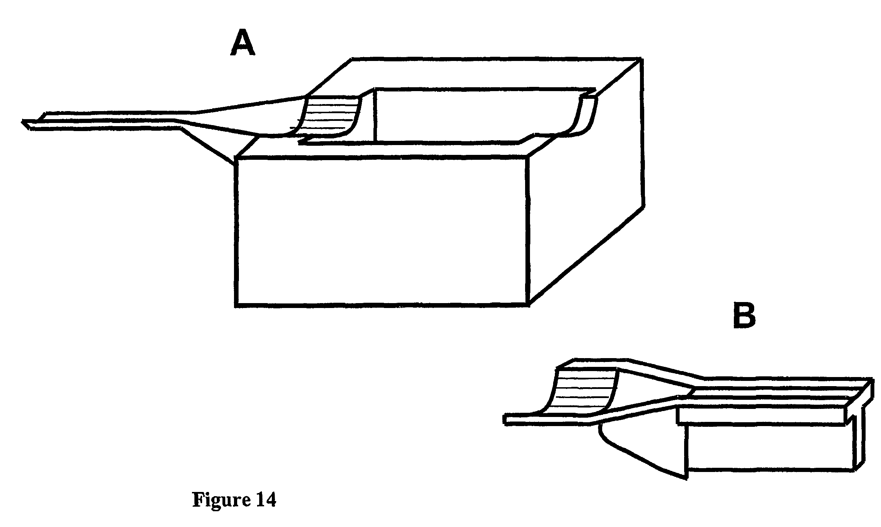

Figure 14 allows accurate positioning of tissue with regard to the locating means.

Following dissection, isolated tissue can be difficult to manipulate as it is flaccid in nature. This is especially true with respect to the accurate positioning of the tissue with regard to the locating means, which may comprise a wire holder, a cannula or the like. To obviate this disadvantage and as shown in Figure 14 immobilising and positioning the tissue can help alignment with the locating device. By placing the tissue structure gently using forceps upon a layer comprising one or more proteins selected from the following: collagen, elastin, gelatin, fibronectin, fibrinogen, vitronectin the tissue can be held in place.

The protein layer can be placed upon a polypropylene or polyethylene surface. Preferably, the plastic surface comprises the inner aspect of half of a conical funnel, ensuring a concave surface upon which to place the tissue. As can be seen in Figure 14, two such funnels are then placed in opposition upon a rigid bath, with the mouths of the funnels facing each other. One funnel (A) is fixed as part of the bath, the second funnel (B) is placed on the bath, preferably attached to a rail that allows movement towards or away from the first funnel in the horizontal plane.

Further, the accurate positioning of the tissue with respect to the chosen locating device is ensured by. gently placing one end of the tissue in the neck of the fixed conical funnel (A) at a point that equals the external diameter of the tissue. The second funnel (B) is moved along the horizontal plane until the end of the tissue in the neck of the conical funnel again lies above the point in the funnel that equals the external diameter of the tissue. The tissue is then gently placed on the protein layer of the moveable second funnel (B).

The locating device can be guided firstly through the narrow portion of the funnel and then through the immobilised tissue.

Various examples of experiments will now be described. The experiments were all performed with a modified pressure myograph (P110, Danish MyoTech, Aarhus, Denmark) with four chambers in each module and multi-reservoir capabilities. A Type 205U/CA multi-channel cassette pump (Watson-Marlow, Camlab, Cambridge, UK) was also used.

The suppliers' instructions were used for operation and preparation of the myograph and pump, modified where appropriate for implementation of the present measurement protocol.

**Examples**

**Example 1. Absorption spectroscopy of agonist-induced vasoconstriction and vasodilation in rat mesenteric arteries.**

3rd order mesenteric arteries from normotensive male Wistar rats were set up in two perfusion chambers in normal physiological salt solution (PSS), at 37°C and gassed with 95%$O_2$/5%$CO_2$. The vessels were pressurised to 60 mmHg and allowed to equilibrate for 30 minutes. Thereafter, one vessel acted as a control and the other vessel received increasing concentrations of the thromboxane A2 mimetic, U46619. Transmission spectra were recorded at one second intervals and analysed according to the methods described above.

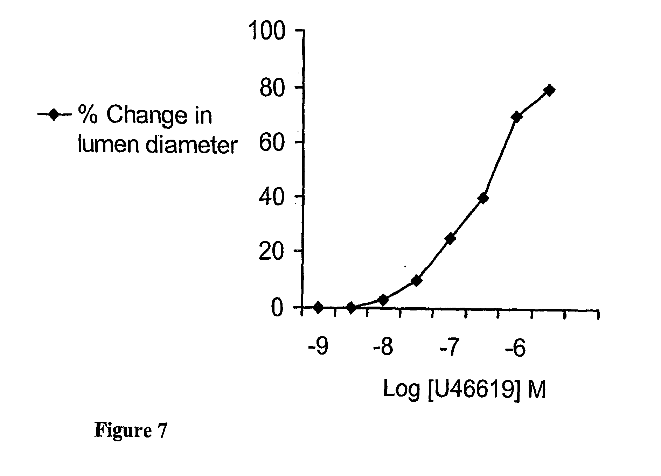

U46619-induced vasoconstriction was detected via shifts in the transmission spectra due to changes in lumen diameter. The optical extinction model described above was used to accurately and continuously measure lumen diameter. This data can then be expressed as graph of concentration (of agonist) versus response (% change in lumen diameter) (figure 7).

**Experiment 2. Absorption spectroscopy of rat arteries.**

Third order mesenteric resistance arteries were obtained from adult female normotensive Wistar and hypertensive Goldblatt (one kidney-one clip) rats 8 weeks following the induction of hypertension. Twenty arterial samples (10 normotensive; 10 hypertensive) were studied from 16 animals (6 eight-week normotensive, 5 eight-week hypertensive, 3 four-week normotensive, and 2 four-week hypertensive) . Dissected arterial segments were stored in cold saline solution prior to mounting in the myograph.

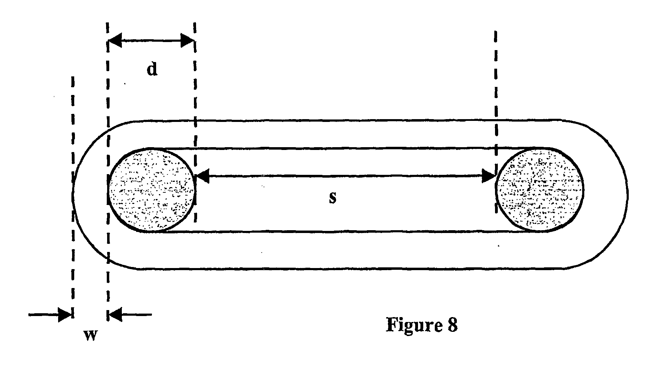

Ring preparations were mounted on two stainless steel wires (40μm in diameter) in physiological salt solution gassed in 95%$O_2$/5%$CO_2$ and maintained at 37°C to a pH of 7.4. The wires were secured onto the myograph mounting heads to enable the internal diameter of the vessel to be controlled directly via an automated linear stage attached to one mounting head. Morphological measurements of media thickness (w), wire diameter (d) and wire separation (s) were made using light microscopy and a filar calibrated eyepiece with the vessels under conditions of minimum tension (Figure 8). The average of three values taken at locations equidistant along each vessel was used. Assuming a circular cross-section under physiological conditions, effective internal circumference (C), lumen diameter (L) and cross-sectional area of the vessel wall (A) were calculated using the following equations:

$$C = d (2 + P) + 2s$$

$$L = C , P$$

$$A = Pw (L + w)$$

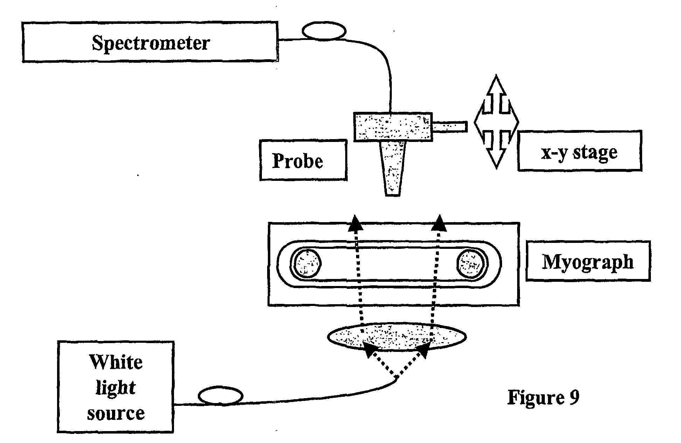

Spectroscopy measurements were achieved by a simple optic fibre-coupled spectrometer integrated into the Mulvany myograph via a probe terminating above the vessel. Light from a tungsten halogen source (150W) was channelled via an optical fibre and collimating lens to the myograph (Figure 9). The fibre optic probe (tip: 1mm diameter; glass fibre: 50 $\mu$m core diameter) was positioned above the vessel by means of a probe assembly clamped to horizontal (resolution 0.01mm) and vertical (resolution 0.05mm) linear stages to enable accurate alignment of the transmission system. A diode array spectrometer coupled to the probe recorded at 2-3 seconds intervals as the lumen diameter was stretched in 50 $\mu$m steps from 100 $\mu$m to 350 $\mu$m. Transmission spectra were obtained from two probe positions: one collected with a stationary probe head and the other with the probe head moved 20 $\mu$m for each 50 $\mu$m horizontal stretch. A total of 659 spectra were used in the subsequent analysis. A standard normalisation method was used to transform the raw spectra into transmission spectra, defined as the ratio between the background subtracted raw arterial spectrum and the white light spectrum. Analysis of transmission spectra Assuming that observed features distinguishing normal and hypertensive vessels reflect an underlying compositional change in the vessel wall, a quantitative analysis of the spectra could in principle have been performed by multivariate analysis of the constituents. However, since at this stage the existence and nature of such compositional changes has not been established an empirical approach was preferred in order to establish the extent and significance of the main spectral features. Given the overall shape of the transmission spectra (T) under examination, a compact representation was possible using the logistic functional:

$$T = \sum_i \frac{a_i}{1 + \exp(-b_i [ \lambda - \lambda_i ])}$$

A quasi-Newton algorithm was used to fit this non-linear function to the observed spectra. Sufficiently accurate data fits were obtained (RMS error = 3%) using only two terms from this equation which were adopted for the analysis reported here.

## Results

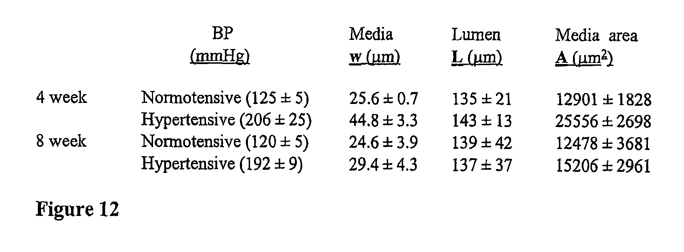

The mean dimensions of the arterial specimens measured by microscopy are given in Figure 12.

The presence of vascular growth was apparent in both hypertensive age groups. The 4-week hypertensive animals showed a marked increase of 98% in media cross-sectional area (65% in media:lumen ratio) compared to their paired normotensive controls. The vessels from the 8-week hypertensive group also showed increases in these parameters (20% in media cross-sectional area; 20% in media:lumen ratio). The data for the 4-week group was skewed since one single animal developed an unusually high pressure (231mmHg) and marked structural growth. Since the purpose of this study was to ascertain the usefulness of spectroscopy, and not to investigate aspects of this particular model of hypertension, these data were subsequently extremely useful.

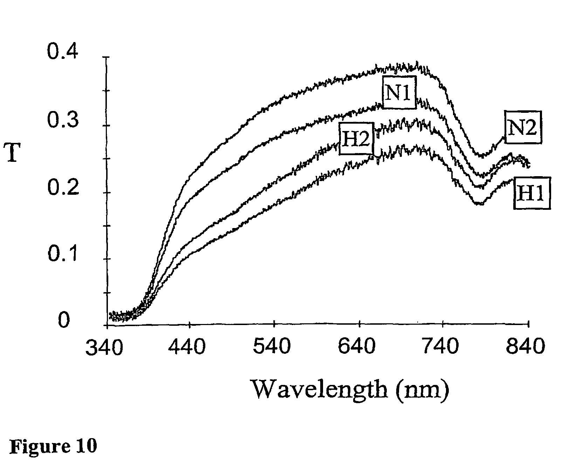

Transmission spectra (resolution = 1 nm) derived from the vessels of 4-week animals show a clear separation between normotensive and hypertensive vessels since changes in vessel wall thickness are reflected in vertical shifts of the spectra (Figure 10). The difference observed between the 8-week groups were not as marked due to the thicker walls exhibited by the 4-week group. Differential increase of the arterial lumen (N1 to N2 and H1 to H2) affected the observed absorption spectra in the vertical plane confirming the correlation between wall thickness and absorption spectra. More subtle features distinguishing hypertensive and normotensive vessels were observed including a high absorption characteristic in the blue-green region of the spectrum (400nm - 550nm, Figure 10). Given the consistency of these observations, a future detailed quantitative analysis is appropriate to ascertain the source of these data.

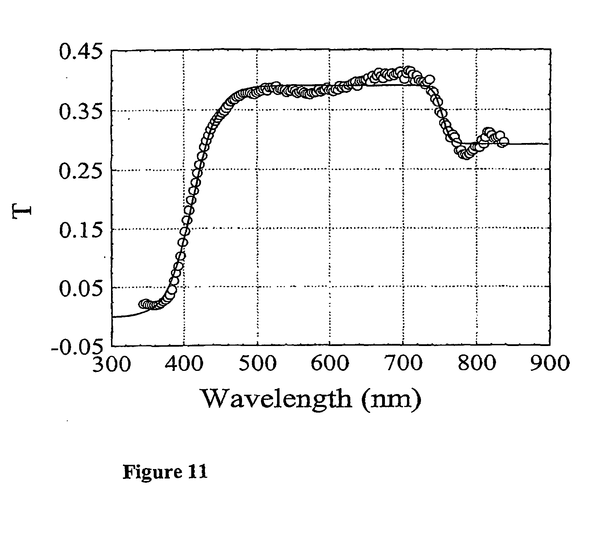

A typical transmission spectrum and its empirical model are shown in Figure 11 for which the model parameters are: $a_1 = 0.39$; $a_2 = 0.098$; $b_1 = 0.057nm^{-1}$; $b_2 = 0.179nm^{-1}$; $\lambda_1 = 412nm$; $\lambda_2 = 753nm$. Each of these parameters exhibits a different sensitivity with respect to the data fitting procedure and therefore the precision of the parameter estimates was individually assessed. This assessment has been performed elsewhere and the conclusion of that analysis leads to the following worst case estimated errors on the determination of model parameters: $a_1$ ($\pm$ 0.02) ; $a_2$ ($\pm$0.03); $b_1$ ($\pm$ 0.006); $b_2$ ($\pm$ 1.5); $\lambda_1$ ($\pm$ 4); $\lambda_2$ ($\pm$ 44). These errors are much larger than those of the majority of the spectra examined and are quoted to allow a conservative interpretation of the results. It should also be stressed that these errors are derived from the limited data sets provided and should only be used as a guide in the consideration of absolute errors.

## Comparing spectral and morphological results.

Both hypertensive age groups showed media growth trends. The linear correlation coefficients obtained from a comparison between the transmission spectral features ($a_1$, $a_2$, $b_1$, $b_2$, $\lambda_1$, $\lambda_2$) and the param-

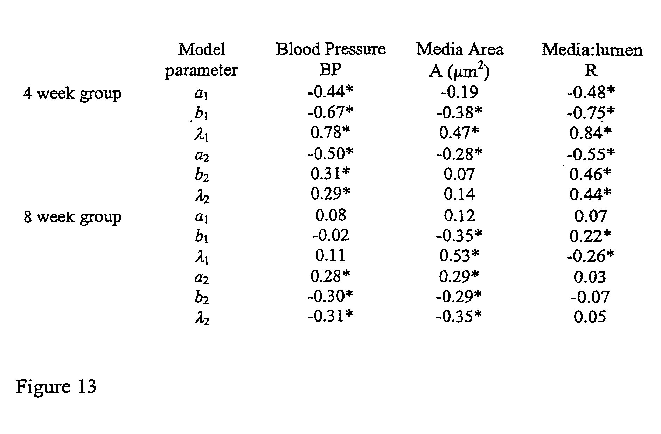

eters which are conventionally used in the study and clinical definition of hypertension (media:lumen ratio (R), media cross sectional area (A) and blood pressure (BP)) are detailed in Figure 13. A number of statistically significant correlations exist between the features extracted from spectroscopy arid those conventionally used to define hypertension, with those of greater significance and closer approach to linearity being found in the 4 week group. The media:lumen ratio is expected to reflect changes in vessel size associated with anatomical variability and media growth, and this should in turn be reflected in a rescaling of the transmission spectra. These effects are indeed implied by the significant correlation between R and the scaling factors for the 4-week group. In the 8-week group, with a less marked difference in structure, the number of correlations is reduced however significant changes can still be detected in two scaling factors. Indeed, the most significant correlations occur between $b_1$, $\lambda_1$ and BP, R. Since BP and R are themselves strongly correlated in this (correlation coefficient = 0.98 for 4 week group) and many other studies their relationship to the observed spectral features is the most important result of this present study. Since these spectral features relate to the logistic function (equation above) which models the average spectral shape in the 350-550nm range, a connection is established between the presence of hypertension and the existence of differential absorption in the blue-green region of the spectrum.

The following examples relate to hypothetical measurements which could be made using the apparatus and method of the present invention.

**Example 1. Absorption spectroscopy of proximal and distal human subcutaneous arteries from critical limb ischaemia patients.**

Human subcutaneous arteries from critical limb ischaemia patients are set up in the perfusion unit and perfused with normal PSS. Vessels proximal to the site of limb ischaemia are compared to vessels distal to the site of limb ischaemia. The absorption spectra from each type of vessel is used to examine differences in media:lumen ratio, cross-sectional area of the vessel wall, and wall thickness.

The results demonstrate whether ischaemic (I) arteries have markedly different absorption spectra from proximal non-ischaemic (N) vessels, reflecting any differences in their wall thickness, media:lumen ratio and lumen diameters.

The apparatus may also be used for transfection experiments. During this process the blood vessel will be exposed to any drug or vector chosen by the investigator. If this is a vector for transfecting genetic material then the material will be carried into the vascular tissue if the pressure and flow settings are chosen appropriately. Once there, the cells of the blood vessel will start to produce the specific protein coded by the genetic material. These proteins will make their way to the cell membrane and lodge there. The time required for this process depends on the vector and particular system being encouraged. Following a suitable transfection period (determined by the investigator and programmed at the beginning of the test) the perfusion reservoir is changed to a reservoir containing an agonist of choice, selected to be appropriate to stimulate the protein induced during transfection. On contact with the proteins in the tissue, the agonist will induce a functional response in the tissue (usually vasoconstriction or vasodilation, possibly changes in wall structure). Any such change will be reflected in an alteration in the optical properties of the vascular tissue.

**Example 2. Transient transfection of NOS synthase gene in NOS-knockout mice using LipofectAMINE reagent and functional detection of transfection by absorption spectroscopy.**

Mouse $1^{st}$ order mesenteric arteries are dissected out and maintained in Optimem I supplemented with 10% foetal bovine serum. cDNAs (for NOS) inserted into appropriate plasmids are mixed with LipofectAMINE according to the manufacturers instructions. Two arteries are set up in separate perfusion chambers; the first tissue (A) acts as a control (receives LipofectAMINE and plasmids without the cDNA insert), the second tissue (B) receives LipofectAMINE with plasmids containing the NOS cDNA insert. The arteries are exposed to the appropriate transfection solutions for 8 hours.

**Determination of NOS cDNA transfection efficiency.**

After 8 hours, the unit switches automatically and perfuses the vessels with normal PSS. Analysis of the lumen diameter via absorption spectroscopy begins, providing continual information on the contractile state of the artery.

Functional detection of NOS activity is achieved by way of a two-stage protocol. Firstly, concentration-response curves to ACh are conducted in both vessels. During the concentration-response curve, changes in the state of the blood vessels are continuously monitored by absorption spectroscopy. ACh selectively activates expressed NOS synthase and causes release of the vasodilator nitric oxide (NO). Therefore, the presence of NOS is demonstrated by vasodilation of the blood vessel in response to ACh. The control vessel should display little vasodilation to ACh, as NOS cDNA was not transfected in this tissue.

Both vessels are then perfused with normal PSS for thirty minutes. To ensure that ACh-induced vasodilation is due to NOS activation and not other unidentified factors, the second stage of the protocol is initiated. The concentration response curve to ACh is repeated in the presence of the selective inhibitor of NOS, L-NAME. If ACh produced vasodilation via NOS activation in the first stage of the protocol then L-NAME should abolish further responses to ACh.

**Example 5. Transfection of gastrointestinal tract from ORL-1 knockout mice with ORL cDNA using adenoviral vectors.**

Two 3cm segments of mouse gastrointestinal tract are set up in perfusion chambers and perfused with normal PSS. cDNAs for the ORL-1 receptor are ligated into an adenovirus transfer plasmid with the cytomegalovirus promotor. The virus is diluted in Optimem I and then perfused through one segment of gastrointestinal tract for up to 2 hours, the other segment acts as a control and receives the virus without the ORL-1 cDNA.

After the transfection period, functional detection of the expressed ORL-1 receptor is achieved by spectroscopic detection of contractions upon perfusion of the selective ORL-1 agonist, nociceptin. The control tissue should display no response. To ensure that the actions of nociceptin are through the ORL-1 receptor, the addition of nociceptin is repeated in the presence of a selective ORL-1 antagonist, such as naloxone. Therefore, this antagonist blocks responses involving the ORL-1 receptor, and nociceptin should have no effect.

**Claims**

1.  An in vitro method of detecting a change in the physical dimensions of a biological structure, the method comprising the steps of:

    illuminating a biological structure with light of a first wavelength and light of at least one further wavelength;

    detecting the intensity of light of each wavelength transmitted by the biological structure in at least first and second physical states; and

    comparing the intensity of transmitted light of each wavelength with a reference light intensity of the same wavelength, to determine a change in the absorption of the biological structure, said change in the absorption corresponding to a change in physical dimensions,

    wherein said method includes the step of distinguishing measured intensities of each wavelength of light used.

2.  A method as claimed in Claim 1 wherein the step of detecting transmitted light comprises the step of detecting the intensity of transmitted light across substantially the whole diameter of the biological structure.

3.  A method as claimed in Claim 1 or claim 2 wherein wavelengths are selected such that in one of the wavelengths the biological structure is essentially opaque, whilst in another of the wavelengths the biological structure is semi-transparent.

4.  A method as claimed in any of the proceeding claims wherein the illumination, detection and comparison steps with second or subsequent wavelengths are performed subsequently to the steps conducted with the first wavelength.

5.  A method as claimed in any of Claims 1 to 3 wherein the illumination, detection and comparison steps with second or subsequent wavelengths are performed simultaneously with the first wavelength.

6.  A method as claimed in Claim 5 wherein separate detectors are used.

7.  A method as claimed in any one of the preceding claims wherein the detected combined intensity is subjected to post-processing to separate the various wavelength components.

8.  A method as claimed in any preceding Claim wherein the reference light intensity corresponds to the intensity of light measured when no biological sample is illuminated.

9.  A method as claimed in any preceding Claim wherein the reference light intensity corresponds to the measured intensity of light transmitted by the biological sample in a different physical state.

10. A method as claimed in any of the preceding Claims wherein the changes in the physical dimensions of a biological structure are measured over time, the method comprising the steps of:

    illuminating a biological structure at first and second time points with light of a first wavelength;

    detecting the intensity of light transmitted by the biological structure at first and second time points; and

    comparing the intensity of transmitted light at the first time point with the intensity of transmitted light at the second time point to determine a change in the intensity of transmitted light, said intensity change being indicative of a change in physical dimensions of the biological structure.

11. A method as claimed in Claim 10 further comprising the step of altering the local environment of the biological structure between said first and second time points.

12. A method as claimed in Claim 11 wherein the step of altering the local environment includes addition or removal of agonists, hormones, potential drugs and/or test samples to the vicinity of the biological structure, and/or direct physical stimulation of the structure and/or transfecting the biological structure with exogenous nucleic acid.

13. Use of a method as claimed in Claim 12 to determine the effect of a gene or similar nucleic acid structure, or the potential stability of a transfection vector or of the biological structure for transfection.

14. A method as claimed in any of Claims 1 to 12 wherein peptide or protein structures are added to the biological structure, to determine the effects of such peptides or proteins directly, without the requirement for expression of a transfected gene.

15. An apparatus for detecting changes in physical dimensions of biological structures, the apparatus comprising at least one light source (46) for generation of light of a plurality of wavelengths a light detector (60) capable of detecting the intensity of light transmitted by said biological of structures at each of said wavelengths means for separating detected light of each of said wavelengths, means (40) for locating a biological structure between the source and the detector, and data processing means (62) for recording and analysing detected light which has been transmitted by the biological structure to detect and analyse changes in characteristics of the transmitted light to determine changes in the physical dimensions of the biological structure.

16. An apparatus as claimed in Claim 15 wherein the at least one light source comprises a plurality of light sources each of which generates light of a single wavelength.

17. An apparatus as claimed in Claim 15 wherein the at least one light source comprises at least one light source which generates light of a plurality of wavelengths.

18. An apparatus as claimed in any of Claims 15 to 17 wherein the light detector comprises a CCD device, a photoresistor or a photodiode.

19. An apparatus as claimed in any of Claims 15 to 18 wherein the means for separating detected light of multiple wavelengths comprises means chosen from post-detection means or pre-detection means, or filter means for allowing only light of a single wavelength to reach the detector.

20. An apparatus as claimed in any of Claims 15 to 19 which further comprises means for aligning generated and detected light.

21. An apparatus as claimed in Claim 20 wherein the means for aligning comprises aligned optical fibres, at least one of which carries generated light, and at least one of which carries detected light.

22. An apparatus as claimed in Claim 21 wherein the fibres are connected at one end to a light source or light detector with the other ends of the fibres being in opposed alignment, such that light leaving the source fibre can enter the detection fibre.

23. An apparatus as claimed in Claim 22 which further comprises a plastics or similar optical fibre holder, arranged to hold optical fibres in oppositely aligned relationship.

24. An apparatus as claimed in Claim 23 wherein the holder comprises a bifurcated tube, the tips of each fork of which are in alignment and wherein in use, an optical fibre is insertable into each fork of the holder, with the free ends of the fibres being coterminous with the ends of the forks to ensure the optical fibres are aligned correctly.

25. An apparatus as claimed in any of Claims 15 to 24 wherein the means for locating a biological structure comprises a wire holder, a cannula, or a plastics tube bearing an external circumferential groove; wherein in use, a biological tube structure may be located over the plastic tube, and secured thereto by means of tying a fastener around the groove.

26. An apparatus as claimed in any of Claims 15 to 25 which further comprises means for subjecting the biological structure to fluids and the like.

27. An apparatus as claimed in Claim 26 wherein the means comprises at least one chamber having a fluid inlet and a fluid outlet, in which the biological structure is located.

28. An apparatus as claimed in Claim 27 which comprises one or more fluid reservoirs, for storing circulatory fluid.

29. An apparatus as claimed in Claim 27 or Claim 28 wherein the chamber comprises a fluid pump, for circulating fluid within the chamber, or into and out of the chamber.

30. An apparatus as claimed in Claim 29 wherein the pump is arranged to maintain the chamber at substantially physiological fluid pressure.

**31.** An apparatus as claimed in any of Claims 15 to 30 which further comprises means for introducing substances to the biological structure.

**32.** An apparatus as claimed in Claim 27 in which a plurality of chambers are provided, enabling a plurality of biological structure samples to be studied simultaneously.

**33.** An apparatus as claimed in any of Claims 15 to 32 wherein the data processing means comprises a computer.

**34.** An apparatus as claimed in any of Claims 15 to 33 which comprises temperature regulation means, for regulating the temperature of a biological sample.

**35.** An apparatus as claimed in any of Claims 15 to 34 wherein the means for locating the biological structure comprises or includes a layer of protein which can immobilise the structure.

**36.** An apparatus as claimed in Claim 35 wherein the protein is or is derived from one or more of the group consisting of collagen, elastin, gelatin, fibronectin, fibrinogen and vitronectin.

**37.** Use of a method as claimed in any of Claims 1 to 14 to detect changes in the internal diameter of a tube structure.

**38.** Use of a method as claimed in any of Claims 1 to 14 to measure a biological tube structure such as those chosen from the group including blood vessel, lymphatic vessel, airway, gut, ureter, urethra, seminiferous, tubule, vas deferens and the like.

**Patentansprüche**

**1.** Ein In-vitro-Verfahren zum Feststellen einer Veränderung in den physischen Dimensionen einer biologischen Struktur, wobei das Verfahren die folgenden Schritte beinhaltet:

Ausleuchten einer biologischen Struktur mit Licht einer ersten Wellenlänge und Licht mindestens einer weiteren Wellenlänge,

Feststellen der Intensität des Lichtes jeder Wellenlänge, das von der biologischen Struktur durchgelassen wurde, in mindestens einem ersten und zweiten physischem Zustand und

Vergleichen der Intensität des durchgelassenen Lichtes jeder Wellenlänge mit einer Referenzlichtintensität der gleichen Wellenlänge, um eine Veränderung der Absorption der biologischen Struktur zu ermitteln, wobei die Veränderung der Absorption einer Veränderung physischer Dimensionen entspricht,

wobei das Verfahren den Schritt des Unterscheidens der gemessenen Intensitäten jeder Wellenlänge des verwendeten Lichtes umfasst.

**2.** Verfahren gemäß Anspruch 1, wobei der Schritt des Feststellens von durchgelassenem Licht den Schritt des Feststellens der Intensität von durchgelassenem Licht über im Wesentlichen den gesamten Durchmesser der biologischen Struktur beinhaltet.

**3.** Verfahren gemäß Anspruch 1 oder Anspruch 2, wobei Wellenlängen so ausgewählt werden, dass die biologische Struktur bei einer der Wellenlängen im Wesentlichen opak ist, während die biologische Struktur bei einer anderen der Wellenlängen semitransparent ist.

**4.** Verfahren gemäß einem der vorherigen Ansprüche, wobei die Schritte des Ausleuchtens, Feststellens und Vergleichens bei zweiten oder nachfolgenden Wellenlängen im Anschluss an die bei der ersten Wellenlänge durchgeführten Schritte ausgeführt werden.

**5.** Verfahren gemäß einem der Ansprüche 1 bis 3, wobei die Schritte des Ausleuchtens, Feststellens und Vergleichens bei zweiten oder nachfolgenden Wellenlängen gleichzeitig mit der ersten Wellenlänge ausgeführt werden.

**6.** Verfahren gemäß Anspruch 5, wobei getrennte Detektoren verwendet werden.

**7.** Verfahren gemäß einem der vorherigen Ansprüche, wobei die festgestellte kombinierte Intensität einer nachträglichen Verarbeitung unterzogen wird, um die Komponenten der verschiedenen Wellenlängen zu trennen.

**8.** Verfahren gemäß einem der vorherigen Ansprüche, wobei die Referenzlichtintensität der Intensität des Lichtes entspricht, die gemessen wird, wenn keine biologische Probe ausgeleuchtet wird.

**9.** Verfahren gemäß einem der vorherigen Ansprüche, wobei die Referenzlichtintensität der gemessenen Intensität des Lichtes entspricht, das von der biologischen Probe in einem anderen physischen Zustand durchgelassen wurde.

**10.** Verfahren gemäß einer der vorherigen Ansprüche, wobei die Veränderungen der physischen Dimensionen einer biologischen Struktur im Zeitablauf gemessen werden, wobei das Verfahren die folgenden Schritte beinhaltet:

Ausleuchten einer biologischen Struktur zu einem ersten und zweiten Zeitpunkt mit Licht einer ersten Wellenlänge,

Feststellen der Intensität des von der biologischen Struktur zu einem ersten und zweiten Zeitpunkt durchgelassenen Lichtes und

Vergleichen der Intensität des durchgelassenen Lichtes zu dem ersten Zeitpunkt mit der Intensität des durchgelassenen Lichtes zu dem zweiten Zeitpunkt, um eine Veränderung der Intensität des durchgelassenen Lichtes zu ermitteln, wobei die Intensitätsveränderung ein Hinweis auf eine Veränderung der physischen Dimensionen der biologischen Struktur ist.

11. Verfahren gemäß Anspruch 10, das ferner den Schritt des Modifizierens der örtlichen Umgebung der biologischen Struktur zwischen dem ersten und dem zweiten Zeitpunkt beinhaltet.

12. Verfahren gemäß Anspruch 11, wobei der Schritt des Modifizierens der örtlichen Umgebung das Hinzugeben oder Entfernen von Agonisten, Hormonen, potentiellen Arzneimitteln und/oder Testproben zu/aus dem Umfeld der biologischen Struktur, und/oder direkte physische Stimulation der Struktur und/oder Transfizieren der biologischen Struktur mit exogener Nukleinsäure umfasst.

13. Anwendung eines Verfahrens gemäß Anspruch 12, um die Auswirkungen eines Gens oder einer ähnlichen Nukleinsäurestruktur oder die potentielle Stabilität eines Transfektionsvektors oder der biologischen Struktur für die Transfektion zu ermitteln.

14. Verfahren gemäß einem der Ansprüche 1 bis 12, wobei Peptid- oder Proteinstrukturen zu der biologischen Struktur hinzugegeben werden, um die Auswirkungen solcher Peptide oder Proteine direkt zu bestimmen, ohne dass die Expression eines transfizierten Gens erforderlich ist.

15. Eine Vorrichtung zum Feststehen von Veränderungen physischer Dimensionen biologischer Strukturen, wobei die Vorrichtung mindestens eine Lichtquelle (46) zum Erzeugen von Licht einer Vielzahl von Wellenlängen, einen Lichtdetektor (60), der in der Lage ist, die Intensität des von den biologischen Strukturen auf jeder der Wellenlänge durchgelassenen Lichtes festzustellen, Mittel zum Trennen des festgestellten Lichtes einer jeden Wellenlänge, Mittel (40) zum Lokalisieren einer biologischen Struktur zwischen der Quelle und dem Detektor und Datenverarbeitungsmittel (62) zum Aufzeichnen und Analysieren von festgestelltem Licht, das von der biologischen Struktur durchgelassen wurde, beinhaltet, um Veränderungen der Eigenschaften des durchgelassenen Lichtes festzustellen und zu analysieren, um Veränderungen der physischen Dimensionen der biologischen Struktur zu ermitteln.

16. Vorrichtung gemäß Anspruch 15, wobei die mindestens eine Lichtquelle eine Vielzahl von Lichtquellen, von denen jede Licht in einer einzelnen Wellenlänge erzeugt, beinhaltet.

17. Vorrichtung gemäß Anspruch 15, wobei die mindestens eine Lichtquelle mindestens eine Lichtquelle, die Licht in einer Vielzahl von Wellenlängen erzeugt, beinhaltet.

18. Vorrichtung gemäß einem der Ansprüche 15 bis 17, wobei der Lichtdetektor ein CCD-Bauteil, einen Fotowiderstand oder eine Fotodiode beinhaltet.

19. Vorrichtung gemäß einem der Ansprüche 15 bis 18, wobei die Mittel zum Trennen des festgestellen Lichtes mehrerer Wellenlängen aus Post-Feststellungsmitteln oder Prä-Feststellungsmitteln ausgewählte Mittel oder Filtermittel, die es nur Licht einer einzigen Wellenlänge ermöglichen, den Detektor zu erreichen, beinhalten.

20. Vorrichtung gemäß einem der Ansprüche 15 bis 19, die ferner Mittel zum Ausrichten von erzeugtem und festgestelltem Licht beinhaltet.

21. Vorrichtung gemäß Anspruch 20, wobei die Mittel zum Ausrichten ausgerichtete Lichtleitfasern beinhalten, von denen mindestens eine erzeugtes Licht und mindestens eine festgestelltes Licht trägt.

22. Vorrichtung gemäß Anspruch 21, wobei die Fasern an einem Ende mit einer Lichtquelle oder einem Lichtdetektor verbunden sind und die anderen Enden der Fasern entgegengesetzt ausgerichtet sind, sodass Licht, das aus der Quellenfaser austritt, in die Feststellungsfaser eintreten kann.

23. Vorrichtung gemäß Anspruch 22, die ferner einen aus Kunststoff oder ähnlichem hergestellten Halter für die Lichtleitfasern beinhaltet, der so angeordnet ist, dass er die Lichtleitfasern in entgegengesetzt ausgerichteter Beziehung hält.

24. Vorrichtung gemäß Anspruch 23, wobei der Halter eine gegabelte Röhre beinhaltet, wobei die Spitzen jeder seiner Gabeln ausgerichtet sind und wobei bei Anwendung eine Lichtleitfaser in jede Gabel des Halters einsetzbar ist, wobei die freiliegenden Enden der Fasern an die Enden der Gabeln angrenzen, um eine genaue Ausrichtung der Lichtleitfasern sicherzustellen.

25. Vorrichtung gemäß einem der Ansprüche 15 bis 24, wobei die Mittel zum Lokalisieren einer biologischen Struktur einen Drahthalter, eine Kanüle oder eine aus Kunststoff hergestellte Röhre mit einer äußeren Umfangsrille beinhalten; wobei bei Anwendung eine biologische Röhrenstruktur über der aus Kunststoff hergestellten Röhre lokalisiert und daran mittels einer um die Rille gebundenen Befestigung gesichert werden kann.

26. Vorrichtung gemäß einem der Ansprüche 15 bis 25, die ferner Mittel beinhaltet, um die biologische Struktur Fluiden und ähnlichem auszusetzen.

27. Vorrichtung gemäß Anspruch 26, wobei das Mittel mindestens eine Kammer mit einem Fluideingang und einem Fluidausgang, in der die biologische Struktur angeordnet ist, beinhaltet.

28. Vorrichtung gemäß Anspruch 27, die einen oder mehrere Fluidbehälter zum Speichern von Zirkulationsfluid beinhaltet.

29. Vorrichtung gemäß Anspruch 27 oder Anspruch 28, wobei die Kammer eine Fluidpumpe zum Zirkulieren von Fluid innerhalb der Kammer oder in die Kammer hinein und aus ihr heraus beinhaltet.

30. Vorrichtung gemäß Anspruch 29, wobei die Pumpe so angeordnet ist, dass sie die Kammer auf einem im Wesentlichen physiologischen Fluiddruck hält.

31. Vorrichtung gemäß einem der Ansprüche 15 bis 30, die ferner Mittel zum Einführen von Substanzen in die biologische Struktur beinhaltet.

32. Vorrichtung gemäß Anspruch 27, in der eine Vielzahl an Kammern bereitgestellt ist, die das gleichzeitige Untersuchen einer Vielzahl von Proben biologischer Struktur ermöglichen.

33. Vorrichtung gemäß einem der Ansprüche 15 bis 32, wobei das Datenverarbeitungsmittel einen Computer beinhaltet.

34. Vorrichtung gemäß einem der Ansprüche 15 bis 33, die temperaturregulierende Mittel zum Regulieren der Temperatur einer biologischen Probe beinhaltet.

35. Vorrichtung gemäß einem der Ansprüche 15 bis 34, wobei das Mittel zum Lokalisieren der biologischen Struktur eine Proteinschicht, welche die Struktur immobilisieren kann, beinhaltet oder umfasst.

36. Vorrichtung gemäß Anspruch 35, wobei das Protein eines oder mehrere aus der aus Kollagen, Elastin, Gelatine, Fibronektin, Fibronogen und Vitronektin bestehenden Gruppe ist oder davon abgeleitet ist.

37. Anwendung eines Verfahrens gemäß einem der Ansprüche 1 bis 14 zum Feststellen von Veränderungen des inneren Umfangs einer Röhrenstruktur.

38. Anwendung eines Verfahrens gemäß einem der Ansprüche 1 bis 14 zum Messen einer biologischen Röhrenstruktur wie die aus der Blutgefäße, lymphatische Gefäße, Luftwege, Darmkanäle, Harnleiter, Harnröhren, Hodenkanälchen, Samenleiter und dergleichen umfassenden Gruppe ausgewählten.

**Revendications**

1. Un procédé in vitro de détection d'un changement dans les dimensions physiques d'une structure biologique, le procédé comportant les étapes de :

   éclairer une structure biologique avec de la lumière d'une première longueur d'onde et de la lumière d'au moins une longueur d'onde supplémentaire ;

   détecter l'intensité de lumière de chaque longueur d'onde transmise par la structure biologique dans au moins un premier et un deuxième état physique ; et

   comparer l'intensité de lumière transmise de chaque longueur d'onde avec une intensité de lumière de référence de la même longueur d'onde, afin de déterminer un changement dans l'absorption de la structure biologique, ledit changement dans l'absorption correspondant à un changement dans des dimensions physiques,

   dans lequel ledit procédé comprend l'étape de distinguer des intensités mesurées de chaque longueur d'onde de lumière utilisée.

2. Un procédé tel que revendiqué dans la revendication 1 dans lequel l'étape de détecter de la lumière transmise comporte l'étape de détecter l'intensité de lumière transmise sur substantiellement le diamètre tout entier de la structure biologique.

3. Un procédé tel que revendiqué dans la revendication 1 ou la revendication 2 dans lequel des longueurs d'onde sont sélectionnées de telle sorte que dans une des longueurs d'onde la structure biologique soit essentiellement opaque, tandis que dans une autre des longueurs d'onde la structure biologique soit semi-transparente.

4. Un procédé tel que revendiqué dans n'importe lesquelles des revendications précédentes dans lequel les étapes d'éclairage, de détection et de comparaison avec une deuxième longueur d'onde ou une longueur d'onde subséquente sont réalisées à la suite des étapes menées avec la première longueur d'onde.

5. Un procédé tel que revendiqué dans n'importe lesquelles des revendications 1 à 3 dans lequel les étapes d'éclairage, de détection et de comparaison avec une deuxième longueur d'onde ou une longueur d'onde subséquente sont réalisées de façon simultanée avec la première longueur d'onde.

6. Un procédé tel que revendiqué dans la revendication 5 dans lequel des détecteurs distincts sont utilisés.

7. Un procédé tel que revendiqué dans n'importe laquelle des revendications précédentes dans lequel l'intensité combinée détectée est soumise à un post-traitement afin de séparer les divers composants de longueur d'onde.

8. Un procédé tel que revendiqué dans n'importe quelle revendication précédente dans lequel l'intensité de lumière de référence correspond à l'intensité de lumière mesurée lorsque aucun échantillon biologique n'est éclairé.

9. Un procédé tel que revendiqué dans n'importe quelle revendication précédente dans lequel l'intensité de lumière de référence correspond à l'intensité mesurée de lumière transmise par l'échantillon biologique dans un état physique différent.

10. Un procédé tel que revendiqué dans n'importe lesquelles des revendications précédentes dans lequel les changements dans les dimensions physiques d'une structure biologique sont mesurés sur une période de temps, le procédé comportant les étapes de :

   éclairer une structure biologique à un premier moment et un deuxième moment dans le temps avec de la lumière d'une première longueur d'onde ;

   détecter l'intensité de lumière transmise par la structure biologique à un premier moment et un deuxième moment dans le temps ; et

   comparer l'intensité de lumière transmise au premier moment dans le temps avec l'intensité de lumière transmise au deuxième moment dans le temps afin de déterminer un changement dans l'intensité de lumière transmise, ledit changement d'intensité étant indicatif d'un changement dans des dimensions physiques de la structure biologique.

11. Un procédé tel que revendiqué dans la revendication 10 comportant de plus l'étape de modifier l'environnement local de la structure biologique entre ledit premier moment et ledit deuxième moment dans le temps.

12. Un procédé tel que revendiqué dans la revendication 11 dans lequel l'étape de modifier l'environnement local comprend l'ajout ou le retrait d'agonistes, d'hormones, d'éventuels médicaments et/ou d'échantillons d'essai au voisinage de la structure biologique, et/ou la stimulation physique directe de la structure et/ou transfecter la structure biologique avec un acide nucléique exogène.

13. Utilisation d'un procédé tel que revendiqué dans la revendication 12 pour déterminer l'effet d'un gène ou d'une structure d'acide nucléique similaire, ou la stabilité éventuelle d'un vecteur de transfection ou de la structure biologique pour la transfection.

14. Un procédé tel que revendiqué dans n'importe lesquelles des revendications 1 à 12 dans lequel des structures de peptides ou de protéines sont ajoutées à la structure biologique, afin de déterminer les effets de tels peptides ou protéines directement, sans que l'expression d'un gène transfecté soit requise.

15. Un appareil destiné à détecter des changements dans des dimensions physiques de structures biologiques, l'appareil comportant au moins une source lumineuse (46) pour la génération de lumière d'une pluralité de longueurs d'onde, un détecteur de lumière (60) capable de détecter l'intensité de lumière transmise par lesdites structures biologiques à chacune desdites longueurs d'onde, un moyen destiné à séparer de la lumière détectée de chacune desdites longueurs d'onde, un moyen (40) destiné à situer une structure biologique entre la source et le détecteur, et un moyen de traitement de données (62) destiné à enregistrer et analyser de la lumière détectée qui a été transmise par la structure biologique pour détecter et analyser des changements dans des caractéristiques de la lumière transmise afin de déterminer des changements dans les dimensions physiques de la structure biologique.

16. Un appareil tel que revendiqué dans la revendication 15 dans lequel cette au moins une source lumineuse comporte une Pluralité de sources lumineuses qui génèrent chacune de la lumière d'une longueur d'onde unique.

17. Un appareil tel que revendiqué dans la revendication 15 dans lequel cette au moins une source lumineuse comporte au moins une source lumineuse qui génère de la lumière d'une pluralité de longueurs d'onde.

18. Un appareil tel que revendiqué dans n'importe lesquelles des revendications 15 à 17 dans lequel le détecteur de lumière comporte un dispositif DCC, une photorésistance ou une photodiode.

19. Un appareil tel que revendiqué dans n'importe lesquelles des revendications 15 à 18 dans lequel le moyen destiné à séparer de la lumière détectée de multiples longueurs d'onde comporte un moyen choisi parmi des moyens de post-détection ou des moyens de pré-détection, ou des moyens formant filtre destinés à ne laisser que de la lumière d'une longueur d'onde unique atteindre le détecteur.

20. Un appareil tel que revendiqué dans n'importe lesquelles des revendications 15 à 19 qui comporte de plus un moyen destiné à aligner de la lumière générée et détectée.

21. Un appareil tel que revendiqué dans la revendication 20 dans lequel le moyen destiné à aligner comporte des fibres optiques alignées, parmi lesquelles au moins une transporte de la lumière générée, et au moins une transporte de la lumière détectée.

22. Un appareil tel que revendiqué dans la revendication 21 dans lequel les fibres sont raccordées à une extrémité à une source lumineuse ou à un détecteur de lumière, les autres extrémités des fibres étant en alignement opposé, de telle sorte que de la lumière quittant la fibre source peut entrer dans la fibre de détection.

23. Un appareil tel que revendiqué dans la revendication 22 qui comporte de plus un support de fibres optiques en plastique ou similaire, agencé pour soutenir des fibres optiques dans une relation alignée de façon opposée.

24. Un appareil tel que revendiqué dans la revendication 23 dans lequel le support comporte un tube bifurqué dont les bouts de chaque fourche sont en alignement et dans lequel, lors de l'utilisation, une fibre optique peut être insérée dans chaque fourche du support, les extrémités libres des fibres étant limitrophes des extrémités des fourches pour faire en sorte que les fibres optiques soient alignées correctement.

25. Un appareil tel que revendiqué dans n'importe lesquelles des revendications 15 à 24 dans lequel le moyen destiné à situer une structure biologique comporte un support de fil métallique, une canule, ou un tube en plastique portant une rainure circonférentielle externe ; dans lequel, lors de l'utilisation, une structure formant tube biologique peut être située par-dessus le tube en plastique, et assujettie à celui-ci en attachant un élément de fixation autour de la rainure.

26. Un appareil tel que revendiqué dans n'importe lesquelles des revendications 15 à 25 qui comporte de plus un moyen destiné à soumettre la structure biologique à des fluides et analogue.

27. Un appareil tel que revendiqué dans la revendication 26 dans lequel le moyen comporte au moins une chambre ayant une entrée de fluide et une sortie de fluide, dans laquelle la structure biologique est située.

28. Un appareil tel que revendiqué dans la revendication 27 qui comporte un ou plusieurs réservoirs de fluide, destinés à stocker du fluide en circulation.

29. Un appareil tel que revendiqué dans la revendication 27 ou la revendication 28 dans lequel la chambre comporte une pompe à fluide, destinée à faire circuler du fluide au sein de la chambre ou dans et hors de la chambre.

30. Un appareil tel que revendiqué dans la revendication 29 dans lequel la pompe est agencée pour maintenir la chambre à une pression de fluide substantiellement physiologique.

31. Un appareil tel que revendiqué dans n'importe lesquelles des revendications 15 à 30 qui comporte de plus un moyen destiné à présenter des substances à la structure biologique.

32. Un appareil tel que revendiqué dans la revendication 27 dans lequel une pluralité de chambres sont fournies, permettant à une pluralité d'échantillons de structure biologique d'être étudiés de façon simultanée.

33. Un appareil tel que revendiqué dans n'importe lesquelles des revendications 15 à 32 dans lequel le moyen de traitement de données comporte un ordinateur.

34. Un appareil tel que revendiqué dans n'importe lesquelles des revendications 15 à 33 qui comporte un moyen de régulation de température, destiné à réguler la température d'un échantillon biologique.

**35.** Un appareil tel que revendiqué dans n'importe lesquelles des revendications 15 à 34 dans lequel le moyen destiné à situer la structure biologique comporte ou comprend une couche de protéine qui peut immobiliser la structure.

**36.** Un appareil tel que revendiqué dans la revendication 35 dans lequel la protéine est ou est dérivée d'un ou de plusieurs éléments du groupe consistant en collagène, élastine, gélatine, fibronectine, fibrinogène et vitronectine.

**37.** Utilisation d'un procédé tel que revendiqué dans n'importe lesquelles des revendications 1 à 14 pour détecter des changements dans le diamètre interne d'une structure formant tube.

**38.** Utilisation d'un procédé tel que revendiqué dans n'importe lesquelles des revendications 1 à 14 pour mesurer une structure formant tube biologique telle que celles choisies dans le groupe comprenant vaisseau sanguin, vaisseau lymphatique, voies aériennes, intestin, uretère, urètre, séminifères, tubule, canal déférent et analogue.

EP 1 322 218 B1

**Figure 1**

**Extinction Coefficients**

Reference Profile    Profile Time 1    Profile Time 2

26    $\varepsilon_{oi}$    30    32    *Spectral component i*

$\varepsilon_i(t_1)$    $\varepsilon_i(t_2)$

**Intensity**

28    $\varepsilon_{oj}$    *Spectral component j*

**Figure 2**

40

48

52

44  42

56

46  50  54

58  60

62  64

Figure 3

EP 1 322 218 B1

Figure 4

82

86

84

**Figure 5**

Figure 6

EP 1 322 218 B1

Figure 7

Figure 8

Figure 9

**Figure 10**

Figure 11

| | BP (mmHg) | Media w ($\mu$m) | Lumen L ($\mu$m) | Media area A ($\mu$m$^2$) |
|---|---|---|---|---|
| 4 week | Normotensive (125 ± 5) | 25.6 ± 0.7 | 135 ± 21 | 12901 ± 1828 |
| | Hypertensive (206 ± 25) | 44.8 ± 3.3 | 143 ± 13 | 25556 ± 2698 |
| 8 week | Normotensive (120 ± 5) | 24.6 ± 3.9 | 139 ± 42 | 12478 ± 3681 |
| | Hypertensive (192 ± 9) | 29.4 ± 4.3 | 137 ± 37 | 15206 ± 2961 |

**Figure 12**

EP 1 322 218 B1

EP 1 322 218 B1

|  | Model parameter | Blood Pressure BP | Media Area A ($\mu m^2$) | Media:lumen R |
|---|---|---|---|---|
| 4 week group | $a_1$ | -0.44* | -0.19 | -0.48* |
|  | $b_1$ | -0.67* | -0.38* | -0.75* |
|  | $\lambda_1$ | 0.78* | 0.47* | 0.84* |
|  | $a_2$ | -0.50* | -0.28* | -0.55* |
|  | $b_2$ | 0.31* | 0.07 | 0.46* |
|  | $\lambda_2$ | 0.29* | 0.14 | 0.44* |
| 8 week group | $a_1$ | 0.08 | 0.12 | 0.07 |
|  | $b_1$ | -0.02 | -0.35* | 0.22* |
|  | $\lambda_1$ | 0.11 | 0.53* | -0.26* |
|  | $a_2$ | 0.28* | 0.29* | 0.03 |
|  | $b_2$ | -0.30* | -0.29* | -0.07 |
|  | $\lambda_2$ | -0.31* | -0.35* | 0.05 |

Figure 13

A

B

Figure 14